# EUROPEAN PATENT SPECIFICATION

(11) **EP 0 617 629 B1**
(45) Date of publication and mention of the grant of the patent: **17.04.1996**
(21) Application number: 92925083.5
(22) Date of filing: 06.11.1992
(51) Int. Cl.: A61M 15/00

(54) **INHALATION DEVICE**
INHALATIONSGERÄT
DISPOSITIF D'INHALATION

(30) Priority: 12.11.1991 GB 9123953
(43) Date of publication of application: 05.10.1994
(73) Proprietor: MINNESOTA MINING AND MANUFACTURING COMPANY, St. Paul, Minnesota 55133-3427 (US)
(72) Inventor: HODSON, Peter D., Trowell, Notts NG9 3QX (GB)
(74) Representative: Bowman, Paul Alan
(86) International application number: US9209505
(87) International publication number: WO9309832

(56) References cited:
- EP-A- 0 237 507
- WO-A-92/08509
- GB-A- 1 118 341
- GB-A- 2 041 763
- GB-A- 2 165 159

## Description

### Field of the Invention

This invention relates to dry powder inhalation devices.

### Description of the Related Art

Asthma and other respiratory diseases have long been treated by inhalation of medicament. For many years, the two most widely used and convenient choices of treatment have been inhalation of medicament from a drug solution or suspension in an aerosol propellent from a metered dose pressurized inhaler, or inhalation of powdered drug, generally admixed with a powdered excipient, from a dry powder inhaler. With growing concern being voiced over the strong link between depletion of the earth's ozone layer and chlorofluorocarbon emissions, the use of these materials as aerosol propellants in pressurized inhalers is being questioned and interest in dry powder systems has been stimulated.

Most single and multiple dose dry powder inhalers use either individual premeasured doses of medicament which are inserted into a dispensing chamber prior to use, or they incorporate a bulk powder reservoir from which successive quantities of medicament are transferred to the dispensing chamber. Such inhalers generally comprise an air passage leading from the dispensing chamber which terminates in a patient port adapted to be inserted into the mouth or nasal passage of the patient. Patient inhalation at the patient port generates an air stream through the dispensing chamber which carries particles of medicament into the lungs of the patient.

Examples of such dry powder inhalers are disclosed in U.S. Patent Nos. 2,587,215, 3,669,113, 3,948,264, 3,971,377, 4,046,146, 4,098,273, 4,137,914, 4,147,166, 4,192,309, 4,240,418, 4,674,491, 4,846,168; British Patent Nos. 1,118,341, 1,268,051, 1,526,303, 2,041,763, 2,061,735, 2,165,159, and 2,191,718; European Patent No. 237507 and International Patent No. WO 90/07351.

A problem common to many dry powder systems is the tendency of the powdered medicament to agglomerate. Agglomeration is caused by individual particles of medicament adhering together in a semi-rigid mass, and requires an increased inspiratory effort by the patient to separate and entrain drug particles into the air stream. If the patient is unable to provide sufficient inspiratory effort the extent of drug penetration into the lower airways of the lung will be reduced. Larger agglomerated drug particles (> 10 µm) which result from inefficient aerosolization are not stably entrained into the patient's air stream and prematurely deposit in the mouth or throat region which may lead to unwanted systemic side effects, especially when potent drugs are administered.

It is desirable to utilize the action of the patient's breathing both to deagglomerate and aerosolize the powdered drug, thereby overcoming the coordination problems necessary to synchronize inhalation with means for medicament aerosolization. The efficiency of powder aerosolization, however, is solely determined by the patient's inspiratory effort. Consequently, a patient having difficulty breathing, e.g., during an asthma attack, may possess insufficient inspiratory effort to deagglomerate and aerosolize the medicament and inhale the required dose at a time when the patient has the greatest need for the drug.

Many inhalation devices have attempted to solve the problems attributable to powder agglomeration by incorporating into the device deagglomeration and aerosolization means, e.g., a battery-powered solenoid buzzer, which cause or assist deagglomeration and aerosolization of the powdered medicament by breaking up particle agglomerates entirely independent of the patient's inspiratory effort. Examples of such devices are disclosed in, e.g., U.S. Patent Nos. 3,948,264, 3,971,377, and 4,147,166. The device may be made fully independent of the patient by incorporating a breath actuation mechanism responsive to respiratory flow, which is able to synchronize medicament release with patient inhalation. An example of such a device is disclosed in our copending International Patent Application No. 90/00670 filed on 30th April 1990.

Dry powder inhalers are also known which incorporate features to assist the break up of particle agglomerates in the powder laden air stream.

For example, British Patent No. 1,268,051 and U.S. Patent No. 3,669,113 disclose dry powder inhalers in which a premetered dose of powdered medicament is contained in a capsule and the airflow past the capsule is increased in velocity by means of a constriction in the air passage. British Patent No. 2,165,159 discloses a dry powder inhaler with a storage chamber for powdered drug comprising a constricted region in the air passage in the mouthpiece region.

British Patent Nos. 1,478,138, 1,526,303 and 2,061,735 and U.S. Patent Nos. 3,948,264, 4,046,146, 4,137,914, 4,240,418, and 4,846,168 disclose dry powder inhalers having an angled mouthpiece which forces the powder laden air stream to pass round a bend.

U.S. Patent Nos. 2,587,215 and 4,674,491 and International Patent No. WO 90/07351 disclose dry powder inhalers in which the powder laden air stream is forced to take a fairly tortuous path prior to exiting the mouthpieces of the devices. British Patent Nos. 1,118,341 and 2,191,718 and European Patent No. 237507 disclose dry powder inhalers in which the particle laden airstream is forced to pass round interdigitated baffles or similar in the mouthpiece region.

### Summary of the Invention

It has now been found that by providing an air passage for carrying a powder laden airstream with one or more deagglomeration channels of defined configuration, it is possible to impart sufficient shear forces to particle agglomerates entrained therein to separate them into smaller particles which are more readily inhaled by the patient.

Therefore, according to the present invention there is provided a dry powder inhaler for dispensing powdered medicament comprising a housing defining a chamber for receiving a dose of powdered medicament, one or more air inlets, and a patient port adapted for insertion into the mouth or nasal passage of the patient, constructed and arranged to provide an air passage extending from the air inlet(s) through the chamber to the patient port so that patient inhalation at the patient port generates an airflow through the inhaler which entrains particles of medicament from the chamber for inhalation by the patient, and in which the air passage is provided with one or more deagglomeration channels between the chamber and patient through which the airstream with entrained medicament must pass, a first opening communicating with the dispensing chamber, a second opening communicating with the patient port, preamble of claim 1 based on EP-A-0 237 507, each channel has a substantially constant cross-sectional profile with a cross-sectional area no greater than 40 mm², and intermediate of said first and second openings either:
(i) a single bend of from 70 to 160° wherein the minimum radius of curvature of the center of the bend is no greater than 10 mm, or
(ii) two or more bends each of from 35 to 200°, wherein the minimum radius of curvature of the center of the bends is no greater than 10 mm.

### Detailed Description of the Invention

The present invention utilizes one or more shaped and dimensioned channels to confine the powder carrying airstream leaving the dispensing chamber, thereby imparting shear and wall friction forces to the particles. These forces are particularly efficient at breaking up particle agglomerates into smaller particles which are capable of being inhaled into the human lung. The cross-sectional profile and dimensions of the deagglomeration channel(s) are selected so as to maximize delivery of respirable sized particles (2 to 5 µm) over the whole range of likely inhalation rates, while minimizing factors such as drug deposition. It has been found that a channel of substantially constant cross-sectional profile of up to 40 mm² (inclusive), imparts enough shear and wall friction forces to promote the deagglomeration of entrained powder agglomerates.

The final choice of cross-sectional profile will depend on factors such as the starting state of the powder, the required degree of deagglomeration, the pressure drop desired at a particular flow rate and the space available inside the device. The deagglomeration channel(s) may be formed with any suitable profile with the important proviso that the cross-sectional area perpendicular to the longitudinal axis (airflow direction) of the channel is no greater than 40 mm². It is preferred to avoid shapes with abrupt or angular features which tend to promote excessive powder deposition. Larger channels having a cross-sectional area greater than 40 mm² tend not to cause adequate deagglomeration of powder. Smaller channels, that is, having a cross-sectional area of less than 40 mm² aid deagglomeration by increasing air velocities and turbulence, but they also tend to give rise to greater pressure drops for a given airflow rate, resulting in greater powder deposition and therefore a net reduction in the quantity of respirable powder. Thus, it is preferred that the cross-sectional area of the channel(s) is not greater than 30 mm² and more preferably in the range 15 to 25 mm².

It is important to ensure that the pressure drop which the patient needs to attain to provide a suitable airflow rate for inhalation of the medicament, typically 30 to 60 liters/minute, is not excessively uncomfortable. A pressure drop of, e.g., ≦400 mm water is suitable.

Each channel incorporates either a single bend of from 70 to 160° or two or more bends, each of from 35 to 180° with the proviso that the radius of curvature of the center of the bend or bends is no greater than 10 mm. The radius of curvature of the center of the bend(s) is preferably no greater than 5 mm and more preferably from 1.5 to 5 mm.

When the channel(s) is formed with two or more bends, then the second and subsequent bends may be formed in the same or in different planes and be of the same or opposing handedness to that preceding it. Preferably, the channel(s) is provided with two bends, with the second bend being of opposing or reverse-handedness to that of the first, thereby forming an 'S' bend. Where the inhaler comprises a plurality of deagglomeration channels they are normally of a similar or identical configuration.

The channel(s) preferably have a uniform cross-sectional profile throughout their length. However, variations in the cross-sectional profile are permitted providing they do not substantially alter the airflow through the channel(s).

Although the deagglomeration channel(s) may be interposed at any suitable point in the air passage between the dispensing chamber and patient port, it is preferred to form the channel(s) in or immediately adjacent to the patient port to avoid excessive pressure drops. Therefore, the space available inside the patient port region of the inhaler is an important factor determining the configuration of the deagglomeration channel(s). This space may be limited in an inhaler incorporating a breath actuation mechanism in the form of a movable vane responsive to patient inspiration. Ideally, the deagglomeration channel(s) should occupy a volume of around 10 x 15 x 15 mm.

The overall path length provided by the deagglomeration channel(s), that is, the average distance travelled by the powder laden airstream between the first and second openings, is generally no greater than 4 cm and preferably no greater than 3 cm to avoid excessive powder deposition and pressure drop. The internal surface of the channel(s) may be smooth or it may be patterned, e.g., with grooves angled relative to the direction of the airflow to induce turbulence.

The advantages of the present invention may be summarized as follows:
(a) good deagglomeration performance with a high respirable fraction (typically >35% of the total weight of the powder) and a high respirable dose (e.g., >75 µg);
(b) acceptable pressure drop (e.g., at 60 liters/minute);
(c) small space requirements and ready compatibility with breath actuation mechanisms; and
(d) the deagglomeration channel(s) acts as a guard to reduce the chance of any fragments of broken capsules (in capsule dry powder inhalers) or other extraneous matter from being inadvertently inhaled by the patient.

The invention will now be described by way of example with reference to the accompanying drawings in which:
Figure 1 is a longitudinal section through a dry powder inhaler of the present invention;
Figure 2 is an enlarged view of a portion of the mouthpiece of the dry powder inhaler of Figure 1;
Figure 3 is a sectional view along the line A-A of the mouthpiece of the dry powder inhaler of Figure 1, and
Figures 4 and 5 are sectional views of alternative arrangements of mouthpiece suitable for use with the inhaler of Figure 1.

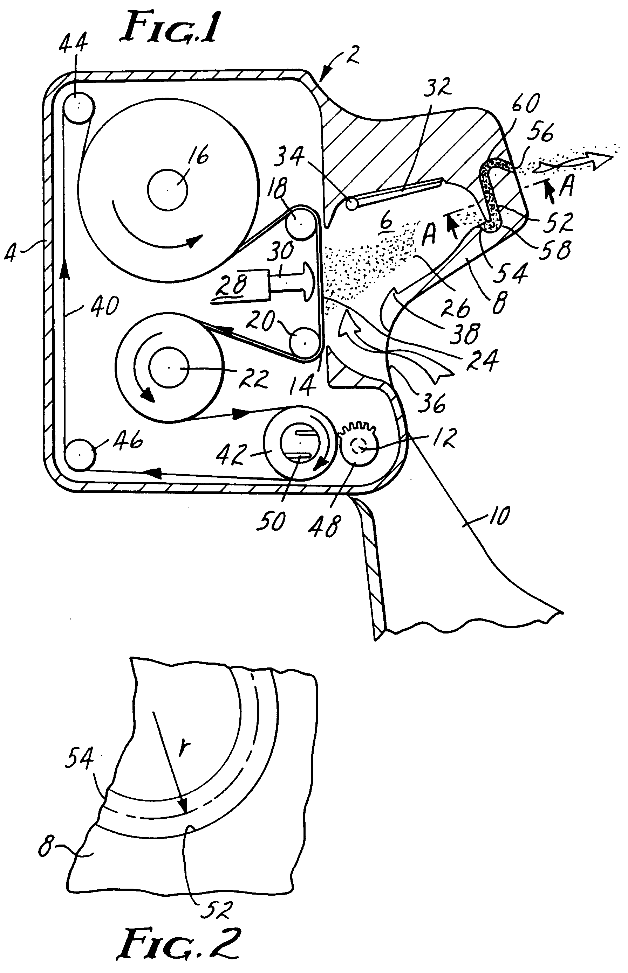

Figure 1 depicts a dry powder inhaler of the present invention.

The device (2) comprises a housing (4) defining a dispensing chamber (6) in communication with a patient port in the form of a mouthpiece (8). A cover (10) is movable about pivot point (12) between a closed position (not shown), which protects the contents of the device from the ingress of moisture and other contaminates, and an open, dispensing position as shown.

The housing (4) contains an elongate carrier (14) which is initially wound on a supply spool (16). From the supply spool (16), the elongate carrier (14) passes round two guide rollers (18 and 20) to a take up spool (22). An area (24) of the elongate carrier (14) between the two guide rollers (18 and 20) is exposed to the dispensing chamber (6). When the device (2) is actuated (as shown), powdered medicament (26) is released from the elongate carrier (14) and entrained in the patient's inspiratory airflow.

The device (2) is operated by the patient opening the cover (10) and inhaling through the mouthpiece (8). This activates three mechanisms, namely: a driving mechanism for advancing the elongate carrier (14); an impaction mechanism which causes the exposed area (24) of the elongate carrier (14) to be impacted, thereby ensuring the release of powdered medicament into the patient's airstream, and a trigger mechanism which ensures that the energy stored in the cocked impaction mechanism is not released until inhalation is detected.

The device (2) comprises means to facilitate the release of the powdered medicament (26) from the elongate carrier (14) in the form of an impaction mechanism (28). After the patient has begun to inhale through the mouthpiece (8), thereby releasing the triggering mechanism, the area (24) of the elongate carrier (14) exposed to the dispensing chamber (6) is struck by a hammer (30) driven by a powerful spring (not shown) to assist the release of medicament (26) into the developing airstream. When cocked, and prior to patient inhalation through the device (2), the hammer (30) is held clear of the elongate carrier (14) by a catch (not shown) which engages the hammer (30) until such time as the trigger mechanism senses patient inhalation. A reset member (not shown) is provided on the cover (10) which, on closure of the device (2), engages and returns the hammer (30) to its original position where it is reengaged by the catch.

The trigger mechanism comprises a movable vane (32) which is pivotally mounted in the mouthpiece (8) at (34) so as to be displaceable when an airflow is generated through the device (2) from the exterior atmosphere to the mouthpiece (8) via air vents (36) (as shown). Displacement of the vane (32) in response to patient inhalation produces an interaction with the catch of the impaction means (28) to release the hammer (30). The vane (32) ensures unidirectional flow of air from the exterior atmosphere to the mouthpiece (8) by being displaceable in the forward direction only. Movement in the reverse direction upon patient exhalation is prevented by stop (38).

The drive mechanism comprises an integral belt (40), the purpose of which is to keep the rotational movement of the supply and take up spools (16 and 22) in precisely the correct relationship to each other and to a control roller (42) regardless of the proportion of the elongate carrier (14) which has been passed between spools. This objective is achieved by the drive belt (40) being in frictional contact with the control roller (42) and with the rear surface of the elongate carrier (14) on each of the spools (16 and 22). In order to achieve the necessary arc of contact between the drive belt (40) and elongate carrier (14) on the spools (16 and 22), the device additionally comprises guide rollers (44 and 46). The elongate carrier (14) is advanced by driving the control roller (42) to cause rotational movement of each spool (16 and 22). The control roller (42) is driven by the act of opening the cover (10) of the device (2) after it has been used by the patient. A drive gear (48) mounted on the pivot (12) of the cover (10) is rotated as the cover (10) is opened and closed. Unidirectional (clockwise) rotation of the control roller (42) is ensured by the provision of a ratchet mechanism (50) which prevents 'wrong-way' rotation when the cover (10) is closed. In this manner, the sequential advancement of unexposed areas (24) of the carrier (14) is coordinated with the simple act of opening and closing the cover (10) of the device (2).

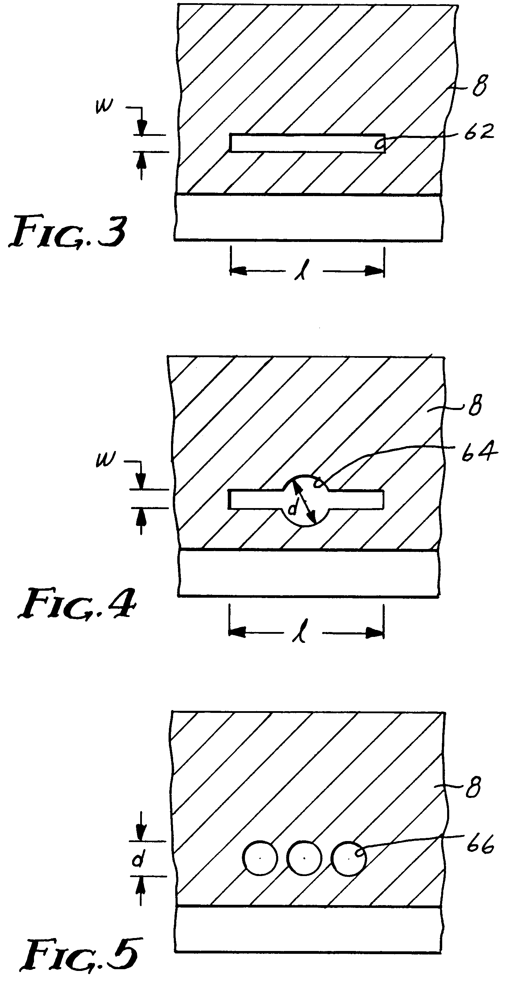

The mouthpiece (8) is provided with a single integrally formed deagglomeration channel (52) which effects the break up of large particle agglomerates released from the carrier (14) into smaller particles of a more respirable size. The deagglomeration channel (52) comprises a first opening (54) communicating with the dispensing chamber (6), a second opening (56) communicating via the mouthpiece with the exterior atmosphere and disposed along its length a first bend (58) of about 150° and a second bend (60) of about 135°. Referring by way of example to Figure 2, which depicts an enlarged view of the first bend (58) of the deagglomeration channel (52), the minimum radius of curvature 'r' of the center of each bend (58 and 60) is no greater than 10 mm, preferably no greater than 5 mm and more preferably between 1.5 and 5 mm (inclusive). It will be appreciated that each bend may not be in the form of a single arc of a circle but may follow a path comprising two or more arcs from circles of different radii conjoined which may include short linear portions. Thus, a bend may follow any desired curve, sinusoidal, parabolic, hyperbolic, etc., providing the other essential parameters of the bend are present. The value of the minimum radius of curvature of the arcs constituting a bend must not be greater than 10 mm. The radius is measured to the centerline of the bend, i.e., mid point between the sidewalls of the channel in the plane of the bend, as shown in Figure 2.

The deagglomeration channel (52) works by constricting the flow of the powder laden airstream from the dispensing chamber, thereby imparting shear and wall friction forces to the particles entrained therein. It is these forces which are responsible for the breakup of the particle agglomerates. Although the deagglomeration channel may have any suitable cross-section which does not promote excessive powder deposition, three particularly preferred profiles are illustrated in Figures 3, 4, and 5. In each case, the total cross-sectional area of the channel when measured at a point perpendicular to the longitudinal axis of the channel should not exceed 40 mm² and is preferably no more than 30 mm².

Referring to Figure 3, the deagglomerator channel is formed with a slot or bar-shaped profile (62) typically having a length (1) of from 8 to 40 mm, preferably 8 to 30 mm and more preferably 8 to 16 mm and a width (w) of from 1 to 5 mm, preferably 1 to 2.5 mm and more preferably 1.2 to 1.6 mm. A slot of 14 mm length with a width of 1.5 mm (total cross-section 21 mm²) was found to release doses of 180 µm, from an inhaler of the type shown in Figure 1 with about 70 µm (39%) of the particles having a size in the range of from 2 to 5 µm (as recorded using a twin stage impinger at a flow rate of 60 1/min).

Figure 4 shows a modification to the deagglomeration channel of Figure 3, in which an opening (54) of greater dimension is formed along the center of the channel in order to reduce the pressure drop experienced by the patient, particularly at high inhalation rates, by providing a central, lower resistance, less turbulent bypass route for air at high flow rates. However, the majority of the powder aerosol still passes close to the walls of the channel where the deagglomerating forces are at their strongest. Typical values for '1', 'w', and 'd' are 14, 1.5, and 4 mm respectively.

Figure 5 illustrates a further arrangement of mouthpiece where the single slot or modified slot of Figures 3 and 4 have been replaced by three equi-size channels of circular cross-section (66). The diameter 'd' of each channel is generally in the range of from 2 to 7 mm, preferably 2 to 5 mm with a typical value of about 3 mm.

## Claims

1. A dry powder inhaler (2) for dispensing powdered medicament (26) comprising a housing (4) defining a chamber (6) for receiving a dose of powdered medicament (26), one or more air inlets, and a patient port (8) adapted for insertion into the mouth or nasal passage of the patient, constructed and arranged to provide an air passage extending from the air inlet(s) (36) through the chamber (6) to the patient port (8) so that patient inhalation at the patient port (8) generates an airstream through the inhaler which entrains particles of medicament (26) from the chamber (6) for inhalation by the patient, the air passage being provided with one or more deagglomeration channel(s) (52) between the chamber (6) and patient port through which the airstream with entrained medicament (26) must pass, a first opening (54) communicating with the dispensing chamber (6), a second opening (56) communicating with the patient port, characterized in that each channel has a substantially constant cross-sectional profile with a cross-sectional area no greater than 40 mm², and intermediate of said first and second openings either:
(i) a single bend of from 70 to 160° wherein the minimum radius of curvature of the center of the bend is no greater than 10 mm, or
(ii) two or more bends each of 35 to 200°, wherein the minimum radius of curvature of the center of the bends is no greater than 10 mm.

2. A dry powder inhaler as claimed in Claim 1 in which the deagglomeration channel(s) has a cross-sectional area no greater than 30 mm².

3. A dry powder inhaler as claimed in Claim 2 in which the deagglomeration channel(s) has a cross-sectional area of from 15 to 25 mm².

4. A dry powder inhaler as claimed in Claim 1 in which the minimum radius of curvature of the center of the bend(s) is no greater than 5 mm.

5. A dry powder inhaler as claimed in Claim 4 in which the minimum radius of curvature of the center of the bend(s) is from 1.5 to 5 mm.

6. A dry powder inhaler as claimed in Claim 1 in which the deagglomeration channel(s) is formed with two or more bends, the second and subsequent bends being of opposite-handedness to that preceding it.

7. A dry powder inhaler as claimed in Claim 1 in which the deagglomeration channel(s) is formed with a first bend of about 150° and a second bend of about 135°.

8. A dry powder inhaler as claimed in Claim 1 in which the overall path length between the first and second openings of the deagglomeration channel(s) is no greater than 4 cm.

9. A dry powder inhaler as claimed in Claim 1 in which the deagglomeration channel(s) has a slot shaped profile of length 8 to 30 mm and width 1 to 2.5 mm.

10. A dry powder inhaler as claimed in Claim 1 in which the deagglomeration channel(s) has a slot shaped profile of length 8 to 16 mm and width 1.2 to 1.6 mm.

11. A dry powder inhaler as claimed in Claim 9 in which the slot has a central aperture to provide a route for air at high(er) flow rates.

12. A dry powder inhaler as claimed in Claim 1 in which the deagglomeration channel(s) has a circular cross-section of diameter 2 to 7 mm.

13. A dry powder inhaler as claimed in Claim 1 in which the deagglomeration channel(s) are formed in or immediately adjacent the patient port.

## Patentansprüche

1. Trockenpulverinhalator (2) zum Ausgeben eines pulverigen Medikaments (26), der aufweist: ein Gehäuse (4), das eine Kammer (6) zum Aufnehmen einer Dosis des pulverigen Medikaments (26) bildet, einen oder mehr Lufteinlässe und einen Patientenanschluß (8), der zum Einführen in den Mund- oder Nasenweg des Patienten geeignet und so aufgebaut und angeordnet ist, daß ein Luftdurchgang vorgesehen ist, der von dem (den) Lufteinlaß (-einlässen) (36) durch die Kammer (6) zum Patientenanschluß (8) so verläuft, daß eine Inhalation des Patienten am Patientenanschluß (8) einen Luftstrom durch den Inhalator erzeugt, der Teilchen des Medikaments (26) aus der Kammer (6) zur Inhalation durch den Patienten mitreißt, wobei der Luftdurchgang versehen ist mit einem oder mehreren Entagglomerierungskanal (-kanälen) (52) zwischen der Kammer (6) und dem Patientenanschluß, die der Luftstrom mit mitgerissenem Medikament (26) passieren muß, einer ersten Öffnung (54), die mit der Ausgabekammer (6) in Verbindung steht, einer zweiten Öffnung (56), die mit dem Patientenanschluß in Verbindung steht, dadurch gekennzeichnet, daß jeder Kanal ein im wesentlichen konstantes Querschnittprofil mit einer Querschnittfläche von höchstens 40 mm² hat sowie zwischen der ersten und zweiten Öffnung entweder:
(i) eine einzelne Biegung von 70 bis 160°, wobei der Mindestkrümmungsradius der Mitte der Biegung höchstens 10 mm beträgt, oder
(ii) zwei oder mehrere Biegungen von jeweils 35 bis 200°, wobei der Mindestkrümmungsradius der Mitte der Biegung höchstens 10 mm beträgt.

2. Trockenpulverinhalator nach Anspruch 1, wobei der (die) Entagglomerierungskanal (-kanäle) eine Querschnittfläche von höchstens 30 mm² hat (haben).

3. Trockenpulverinhalator nach Anspruch 2, wobei der (die) Entagglomerierungskanal (-kanäle) eine Querschnittfläche von 15 bis 25 mm² hat (haben).

4. Trockenpulverinhalator nach Anspruch 1, wobei der Mindestkrümmungsradius der Mitte der Biegung(en) höchstens 5 mm beträgt.

5. Trockenpulverinhalator nach Anspruch 4, wobei der Mindestkrümmungsradius der Mitte der Biegung(en) 1,5 bis 5 mm beträgt.

6. Trockenpulverinhalator nach Anspruch 1, wobei der (die) Entagglomerierungskanal (-kanäle) mit zwei oder mehreren Biegungen ausgebildet ist (sind), wobei die zweite und nachfolgende Biegungen eine entgegengesetzte Richtung zu der vorhergehenden haben.

7. Trockenpulverinhalator nach Anspruch 1, wobei der (die) Entagglomerierungskanal (-kanäle) mit einer ersten Biegung von etwa 150° und einer zweiten Biegung von etwa 135° ausgebildet ist (sind).

8. Trockenpulverinhalator nach Anspruch 1, wobei die gesamte Weglänge zwischen der ersten und zweiten Öffnung des (der) Entagglomerierungskanals (-kanäle) höchstens 4 cm beträgt.

9. Trockenpulverinhalator nach Anspruch 1, wobei der (die) Entagglomerierungskanal (-kanäle) ein schlitzförmiges Profil mit einer Länge von 8 bis 30 mm und einer Breite von 1 bis 2,5 mm hat (haben).

10. Trockenpulverinhalator nach Anspruch 1, wobei der (die) Entagglomerierungskanal (-kanäle) ein schlitzförmiges Profil mit einer Länge von 8 bis 16 mm und einer Breite von 1,2 bis 1,6 mm hat (haben).

11. Trockenpulverinhalator nach Anspruch 9, wobei der Schlitz eine Mittelöffnung hat, um einen Weg für Luft mit hohen (höheren) Strömungsgeschwindigkeiten vorzusehen.

12. Trockenpulverinhalator nach Anspruch 1, wobei der (die) Entagglomerierungskanal (-kanäle) einen kreisförmigen Querschnitt mit einem Durchmesser von 2 bis 7 mm hat (haben).

13. Trockenpulverinhalator nach Anspruch 1, wobei der (die) Entagglomerierungskanal (-kanäle) in dem Patientenanschluß oder unmittelbar daneben ausgebildet ist (sind).

## Revendications

1. Inhalateur (2) à poudre sèche permettant de distribuer un médicament en poudre (26), comprenant un logement (4) définissant une chambre (6) servant à recevoir une dose de médicament en poudre (26), une ou plusieurs entrées d'air et un orifice (8) pour le patient, adapté pour l'introduction dans le passage buccal ou nasal du patient, construit et configuré pour fournir un passage d'air s'étendant depuis l'entrée ou les entrées d'air (36) à travers la chambre (6) jusqu'à l'orifice (8) pour le patient, de sorte que l'inhalation effectuée par le patient, au niveau de l'orifice (8) pour le patient, génère un flux d'air à travers l'inhalateur qui entraîne des particules de médicament (26), depuis la chambre (6), pour l'inhalation effectuée par le patient, le passage d'air étant prévu avec un ou plusieurs canaux de désagglomération (52) entre la chambre (6) et l'orifice pour le patient, à travers lequel le flux d'air, avec le médicament (26) entraîné, doit passer, une première ouverture (54) communiquant avec la chambre de distribution (6), une seconde ouverture (56) communiquant avec l'orifice pour le patient, caractérisé en ce que chaque canal a un profil en coupe transversale à peu près constant avec une superficie de la section ne dépassant pas 40 mm², et comprend en partie intermédiaire desdites première et seconde ouvertures:
(i) soit un seul coude compris entre 70 et 160°, dans lequel le rayon minimum de courbure du centre du coude n'est pas supérieur à 10 mm,
(ii) soit deux coudes ou plus, chacun étant compris entre 35 et 200°, dans lesquels le rayon minimum de courbure du centre des coudes n'est pas supérieur à 10 mm.

2. Inhalateur à poudre sèche comme revendiqué dans la revendication 1, dans lequel le ou les canaux de désagglomération ont une superficie en section transversazle qui n'est pas supérieure à 30 mm².

3. Inhalateur à poudre sèche comme revendiqué dans la revendication 2, dans lequel le ou les canaux de désagglomération ont une superficie en section transversale comprise entre 15 et 25 mm².

4. Inhalateur à poudre sèche comme revendiqué dans la revendication 1, dans lequel le rayon minimum de courbure du centre du ou des coudes n'est pas supérieur à 5 mm.

5. Inhalateur à poudre sèche comme revendiqué dans la revendication 4, dans lequel le rayon minimum de courbure du centre du ou des coudes est compris entre 1,5 et 5 mm.

6. Inhalateur à poudre sèche comme revendiqué dans la revendication 1, dans lequel le ou les canaux de désagglomération sont formés avec deux coudes ou plus, le second coude et les coudes suivants tournant dans le sens opposé à celui qui le précède.

7. Inhalateur à poudre sèche comme revendiqué dans la revendication 1, dans lequel le ou les canaux de désagglomération sont formés avec un premier coude de 150° environ et avec un second coude de 135° environ.

8. Inhalateur à poudre sèche comme revendiqué dans la revendication 1, dans lequel la longueur totale du chemin, entre la première et la seconde ouverture du ou des canaux de désagglomération, n'est pas supérieure à 4 cm.

9. Inhalateur à poudre sèche comme revendiqué dans la revendication 1, dans lequel le ou les canaux de désagglomération ont un profil en forme de fente d'une longueur de 8 à 30 mm et une largeur de 1 à 2,5 mm.

10. Inhalateur à poudre sèche comme revendiqué dans la revendication 1, dans lequel le ou les canaux de désagglomération ont un profil en forme de fente d'une longueur de 8 à 16 mm et une largeur de 1,2 à 1,6 mm.

11. Inhalateur à poudre sèche comme revendiqué dans la revendication 9, dans lequel la fente a une ouverture centrale pour fournir à l'air une route à des débits élevés ou plus élevés.

12. Inhalateur à poudre sèche comme revendiqué dans la revendication 1, dans lequel le ou les canaux de désagglomération ont une section transversale circulaire d'un diamètre de 2 à 7 mm.

13. Inhalateur à poudre sèche comme revendiqué dans la revendication 1, dans lequel le ou les canaux de désagglomération sont formés dans l'orifice pour le patient ou immédiatement adjacents à cet orifice.
